# EUROPEAN PATENT APPLICATION

(11) **EP 3 818 924 A1**
(43) Date of publication of application: **12.05.2021**
(21) Application number: 19207217.1
(22) Date of filing: 05.11.2019
(51) Int. Cl.: A61B 5/00

(54) **APPARATUS FOR MEASURING BIO-SIGNALS**

(71) Applicant: IDUN Technologies AG, 8152 Glattpark (CH)
(72) Inventor: Chardonnens, Séverine, 8152 Glattpark (CH); Bachmann, Simon, 8152 Glattpark (CH); Fümm, Andrea, 8152 Glattpark (CH); Junker, Katja, 8152 Glattpark (CH); Zortea, Lucas, 8152 Glattpark (CH); Vollenweider, Alexandra, 8152 Glattpark (CH)
(74) Representative: Kolster Oy Ab

(57) **Abstract**

An apparatus for measuring bio-signals, the apparatus comprising conductive textile for contacting skin, a holding layer for holding the conductive textile in contact with skin and a conductive connector mechanism operatively connected to the conductive textile, wherein the conductive connector mechanism is integrated in the holding layer

## Description

### TECHNICAL FIELD

The present application relates to an apparatus. More specifically, the present application relates to an apparatus for measuring bio-signals.

### BACKGROUND

Electrodes are used for physiological measurements. Physiological measurements comprise measuring and monitoring a range of physiological parameters.

### SUMMARY

Various aspects of examples of the invention are set out in the claims. The scope of protection sought for various embodiments of the invention is set out by the independent claims. The examples and features, if any, described in this specification that do not fall under the scope of the independent claims are to be interpreted as examples useful for understanding various embodiments of the invention.

According to an aspect of the invention, there is provided an apparatus comprising conductive textile for contacting skin, a holding layer for holding the conductive textile in contact with skin and a conductive connector mechanism operatively connected to the conductive textile, wherein the conductive connector mechanism is integrated in the holding layer.

### BRIEF DESCRIPTION OF THE DRAWINGS

Some example embodiments will now be described with reference to the accompanying drawings:
Figure 1 illustrates an example apparatus; and
Figure 2 illustrates an example exploded view of an apparatus;
Figure 3 illustrates another example exploded view of an apparatus;
Figure 4 illustrates a further example exploded view of an apparatus;
Figure 5 illustrates a yet further example exploded view of an apparatus; and
Figures 6A, 6B, 6C and 6D illustrate different example arrangements.

### DETAILED DESCRIPTION OF THE DRAWINGSS

The following embodiments are exemplifying. Although the specification may refer to "an", "one", or "some" embodiment(s) in several locations of the text, this does not necessarily mean that each reference is made to the same embodiment(s), or that a particular feature only applies to a single embodiment. Single features of different embodiments may also be combined to provide other embodiments.

Example embodiments relate to an apparatus for measuring bio-signals. More particularly, example embodiments relate to an electrode for biopotential data acquisition. According to an example embodiment, the apparatus comprises conductive textile for contacting skin, a holding layer for holding the conductive textile in contact with skin and a conductive connector mechanism operatively connected to the conductive textile, wherein the conductive connector mechanism is integrated in the holding layer.

A bio-signal comprises a signal in a living being that can be continually measured and monitored. A bio-signal may comprise an electrical or non-electrical signal.

An electrical bio-signal may refer to a change in electric current produced by an electrical potential difference between points in living cells, tissues organs or a cell system such as the nervous system. An electrical bio-signal may comprise, for example, electroencephalogram (EEG), electrocardiogram (ECG) or electromyogram (EMG). EEG, ECG and EMG may be measured with a differential amplifier configured to register the difference between two electrodes attached to skin.

A bioelectrode is a mechanism configured to function as an interface between biological structures and electronic systems. Electronic systems may be configured to passively sense, for example, measure or actively stimulate electrical potentials within the biological structures. Bioelectric potentials generated by a living being are ionic potentials that need to be converted into electronic potentials before they can be measured by conventional methods. Electrodes are configured to convert ionic potential to into electronic potential. A bioelectrode is configured to convert body ionic current in a body into electronic current flowing in an electrode.

There are different types of electrodes such as surface electrodes, microelectrodes, internal electrodes and needle electrodes. Surface electrodes are types of electrodes applied to the skin of a subject. Surface electrodes are typically used in ECG, EEG and EMG measurements.

There are different types of electrodes such as wet electrodes, dry electrodes, active electrodes and passive electrodes. Wet electrodes use electrolytic gel material as conductor between the skin and the electrode. Dry electrodes typically comprise a single metal, such as stainless steel, that acts as a conductor between the skin and the electrode. Active electrodes typically comprise a pre-amplification module that allows amplifying a signal before additional is added between the electrode and a system that might capture, process or amplify the signal. Passive electrodes extend the connection from the conductive material to the equipment capturing, processing or amplifying the signal.

However, measuring bio-signals in motion is challenging due to electrodes being sensitive for motion artifact and/or the measurement system being very complex. In particular, current measurement and/or monitoring systems are too complex for consumer applications such as sports wearables.

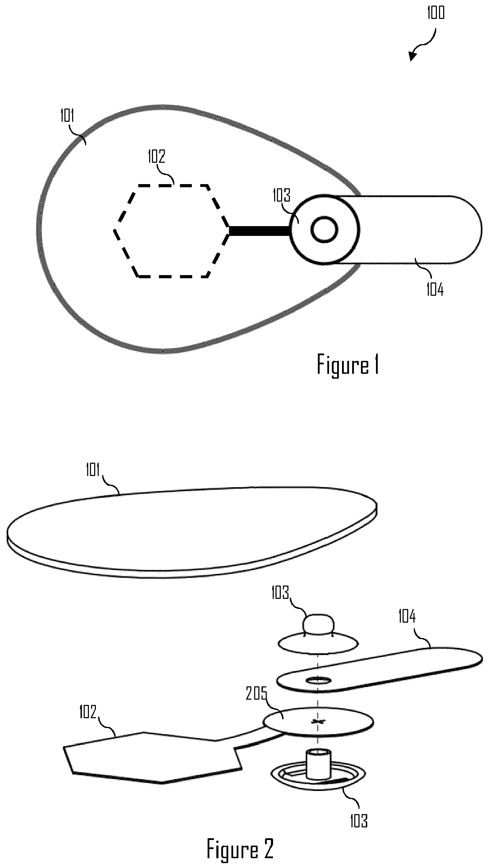

Figure 1 shows an example of an apparatus 100 according to an example embodiment. According to an example embodiment, the apparatus 100 is configured to measure and/or monitor bio-signals on skin. The apparatus 100 in the example of Figure 1 is configured to measure electrical bio-signals such as ECG, EEG and/or EMG. The length of the apparatus 100 may be, for example, 40 to 100 mm, such as 55 mm, 70 mm or 80 mm. The length of the apparatus 100 may also be shorter than 40 mm or longer than 100 mm. The width of the apparatus 100 may be, for example, 20 to 50 mm, such as 40 mm. The width of the apparatus 100 may also be shorter than 20 mm or longer than 50 mm.

According to an example embodiment, the apparatus 100 comprises a dry surface electrode. A dry surface electrode is configured to monitor biopotentials and the dry surface electrode may be applied directly on skin without a need to apply electrolytic gel.

Without limiting the scope of the claims, an advantage of a dry surface electrode is that skin irritations caused by electrolyte gel may be avoided and the time of use may be longer as there is no need to add electrolyte gel to compensate for drying out of the electrolyte gel. A further advantage is that a dry surface electrode enables underwater use as no electrolyte gel is needed.

According to an example the apparatus 100 comprises an electrode in form of conductive textile 102 for contacting skin.

A conductive textile comprises fabric configured to conduct electricity. In other words, conductive textile is configured to act as an electrical conductor. Conductive textiles may be made, for example, by weaving metal strands into a construction of a textile or by conductive yarns which are conductive due to a metal coating.

According to an example embodiment, the conductive textile 102 comprises knitted nylon with a conductive coating. The conductive coating may comprise, for example, silver, gold, platinum or any other suitable metal coating.

The conductive textile may comprise one or more layers of conductive textile. According to an example embodiment, the apparatus comprises a single layer of conductive textile.

According to an example embodiment, the conductive textile 102 is precoated with a conductive polymer layer in order to improve shielding. Shielding may be used for minimizing noise when measuring bio-signals using the apparatus 100. Shielding may be needed, for example, if the conductivity of the conductive polymer layer is low.

Shielding may also be utilized in the manufacturing process. According to an example embodiment, shielding covers the conductive textile 102 such that during manufacturing shielding protects the conductive textile 102. For example, assuming the holding layer 101 is provided by moulding, the shielding protects the conductive textile 102 such that the shielding prevents melt substance such as silicone or thermoplastic elastomer from flowing through the conductive textile 102.

According to an example embodiment, the conductive textile 102 is at least partially integrated in the holding layer 101. The conductive textile 102 may be integrated in the holding layer 101 during manufacturing of the apparatus 100. A conductive textile 102 integrated in the holding layer 101 is illustrated in the example of Figure 1 with a dashed line.

According to an example embodiment, the conductive textile 102 comprises a mesh that is tighter at the centre of the conductive textile 102 and looser at the edges of the conductive textile 102. The density of the mesh may be utilized in the manufacturing process. For example, assuming the holding layer 101 is provided by moulding, the melt substance such as silicone or thermoplastic elastomer may flow through the looser parts of the mesh thereby enabling at least partial integration of the conductive textile 102 in the holding layer 101.

Without limiting the scope of the claims, an advantage of the conductive textile 102 being at least partially integrated in the holding layer 101 is that the conductive textile 102 improves mechanical stability of the apparatus 100.

According to an example embodiment, the shape of the conductive textile 102 comprises a polygon. For example, the shape of the conductive textile may comprise a pentagon, a hexagon, heptagon, octagon or the like.

Without limiting the scope of the claims, an advantage of a polygon conductive textile 102 is that manufacturing the conductive textile 102 may be easier. For example, laser cutting of the conductive textile may be easier. Another advantage of a polygon shape may be that the conductive textile 102 integrates better in the holding layer 101. A further advantage of a polygon shape of the conductive textile 102 is that a polygon may be mechanically stronger than, for example, a round conductive textile.

According to an example embodiment, the apparatus 100 further comprises a holding layer 101 for holding the conductive textile 102 in contact with skin. The holding layer may at least partially extend over the dimensions of the conductive textile. For example, the holding layer may cover the conductive textile such that the holding layer covers a larger area than the conductive textile.

According to an example embodiment, the holding layer 101 is adhesive. An advantage of an adhesive holding layer 101 is that the holding layer 101 is tacky and does not easily slip on skin. Therefore, an adhesive holding layer 101 is configured to provide skin conformity and thereby reduce motion artifact. Reducing motion artifact may comprise reducing motion of the conductive textile 102 relative to skin during movement of a measured object. Reducing motion artifact may also comprise absorbing movement of a measurement cable and/or measurement device. Absorbing movement of a measurement cable and/or measurement device may comprise, for example, providing a soft layer between the conductive textile 102 and the conductive connector mechanism 103.

According to an example embodiment, the holding layer 101 is hydrophobic. As the holding layer 101 extends over the dimensions of the conductive textile 102, a hydrophobic holding layer enables preventing water to enter between the conductive textile 102 and skin.

Without limiting the scope of the claims, an advantage of a hydrophobic holding layer 101 is that the apparatus 100 may be used underwater.

According to an example embodiment, the holding layer 101 comprises a non-conductive polymer layer. According to an example embodiment, the holding layer comprises silicone or thermoplastic elastomer. A thermoplastic elastomer comprises both thermoplastic and elastomeric properties. Further, an advantage of a thermoplastic elastomer is that is it stretchable and also able to return near the original shape.

Without limiting the scope of the claims, an advantage of a silicone holding layer or thermoplastic elastomer is that as the holding layer is tacky and touches skin, the apparatus might not slip so easily. Another advantage of silicone and thermoplastic elastomer is that they are easy to use in manufacturing, for example, by injection moulding.

According to an example embodiment, the holding layer 101 comprises a first part and a second part. The holding layer 101 may be manufactured in two parts in order to make the manufacturing process easier. Manufacturing the holding layer 101 in two parts may also enable integrating electronics into the apparatus 100. Manufacturing the holding layer 101 may comprise, for example, moulding the first layer such that the conductive textile 102 is at least partially integrated in the holding layer 101, adding electronics and overmoulding the second layer such that the electronics is integrated in the holding layer 101. Therefore, manufacturing the holding layer 101 in two parts may enable adding, for example, a flexible printed circuit board (PCB) or other electronics in the apparatus 100.

The holding layer 101 may be manufactured using different processing techniques such as different moulding and/or casting techniques. According to an example embodiment, the holding layer 101 may be manufactured by liquid casting from a polymer/solvent dispersion, injection moulding, thermo moulding, film casting, thermo forming, vacuum forming, reaction injection moulding, blow moulding, extrusion, compression moulding, transfer moulding general thermo moulding or the like.

According to an example embodiment, the holding layer 101 is a moulded layer.

The apparatus 100 may also comprise components that enable manufacturing of the apparatus 100. For example, the apparatus 100 may comprise different kinds of layers and/or structures to support the apparatus and make manufacturing of the apparatus 100 easier.

According to an example embodiment, the apparatus 100 comprises supportive textile. Supportive textile may be provided for ensuring that when manufacturing the holding layer by, for example, moulding, the melt holding layer material flows under the conductive connector mechanism 103 and thereby mechanically and electrically decouples the conductive connector mechanism 103 from skin. The supportive textile is also configured to mechanically support the apparatus 100.

According to an example embodiment, the apparatus 100 further comprises a conductive connector mechanism 103 operatively connected to the conductive textile 102, wherein the conductive connector mechanism 103 is integrated in the holding layer 101.

The conductive connector mechanism 103 is configured to conduct electricity from the conductive textile 102 to a measurement device. The conductive connector mechanism 103 may comprise a mechanical connection or a wireless connection.

According to an example embodiment, the conductive connector mechanism 103 is integrated in the holding layer 101 such that a portion of the conductive connector mechanism 103 extends through the holding layer 101. For example, if the conductive connector mechanism 103 comprises a mechanical connection, a measurement cable may be connected to the conductive connector mechanism 103 extending through the holding layer 101.

According to an example embodiment, integrating the conductive connector mechanism 103 in the holding layer 101 comprises decoupling the conductive connector mechanism from skin.

Without limiting the scope of the claims, an advantage of integrating the conductive connector mechanism is that the conductive connector mechanism does not touch skin and thereby interfere with measuring bio-signals.

According to an example embodiment, the conductive connector mechanism 103 comprises a wire or any other suitable mechanism for conducting electricity from the conductive textile 102 to a measurement device.

According to an example embodiment, the conductive connector mechanism 103 comprises a snap joint. A snap joint may be mechanically connected to the conductive textile 102.

Without limiting the scope of the claims, an advantage of a snap joint is that it is easy to attach and a snap joint is typically compatible with most commercially available cables.

According to an example embodiment, the apparatus 100 comprises flexible electronics such as a flexible printed circuit board (PCB) that is operatively connected to the conductive textile 102 or the conductive polymer with which the conductive textile 102 may be coated. A flexible PCB may enable processing measurement data in the apparatus 100.

According to an example embodiment, the apparatus 100 further comprises a cable detaching mechanism 104. According to an example embodiment, the cable detaching mechanism comprises a lug, for example a plastic lug. In the example of Figure 1, the cable detaching mechanism comprises a lug that a user can hold when detaching a cable connected with the conductive connector mechanism 103.

For example, assuming the conductive connector mechanism is a snap joint extending through the holding layer 101, the cable detaching mechanism may be provided between the conductive textile 102 and the extending portion of the snap joint. According to an example embodiment, the cable detaching mechanism may also be used for labelling.

According to an example embodiment, the conductive connector mechanism 103 is completely integrated in the holding layer 101. For example, if the conductive connection mechanism 103 comprises a wireless connection such as a wireless transmitter, the wireless transmitter may be configured to transmit signals wirelessly to a measurement device without a physical connection.

Figure 2 shows an example of an apparatus 100 in an exploded view according to an example embodiment.

In the example of Figure 2, the apparatus 100 comprises conductive textile 102 for contacting skin, a holding layer 101 for holding the conductive textile 102 in contact with skin, a conductive connector mechanism 103 operatively connected to the conductive textile 102 and a cable detaching mechanism 104. In the example of Figure 2, the apparatus 100 further comprises conductive polymer 205.

According to an example embodiment, a conductive polymer layer 205 may be provided on top of the conductive textile 102 for ensuring that when manufacturing the holding layer by, for example, moulding, the melt holding layer material does not flow through the conductive textile 102.

In the example of Figure 2, the holding layer 101 comprises a single part. However, the holding layer 101 may also comprise a plurality of parts.

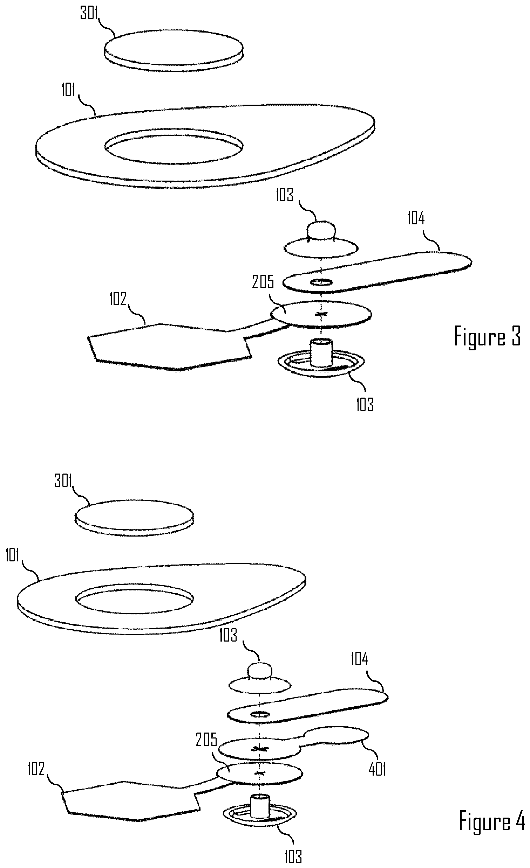

Figure 3 shows an example of the apparatus 100 in an exploded view according to another example embodiment.

In the example of Figure 3, the apparatus 100 comprises conductive textile 102 for contacting skin, a holding layer 101 for holding the conductive textile 102 in contact with skin, a conductive connector mechanism 103 operatively connected to the conductive textile 102, conductive polymer 205 provided on top of the conductive textile 102 and a cable detaching mechanism 104.

In the example of Figure 3, the holding layer 101 comprises a first part 101 and a second part 301. The holding layer 101 may be manufactured in two parts in order to make the manufacturing process easier.

Figure 4 shows an example of the apparatus 100 in an exploded view according to a further example embodiment.

In the example of Figure 4, the apparatus 100 comprises conductive textile 102 for contacting skin, a holding layer 101 for holding the conductive textile 102 in contact with skin, a conductive connector mechanism 103 operatively connected to the conductive textile 102, conductive polymer 205 provided on top of the conductive textile 102 and a cable detaching mechanism 104.

In the example of Figure 4, the apparatus 100 further comprises supportive textile 401 for ensuring that when manufacturing the holding layer by, for example, moulding, the melt holding layer material flows under the conductive connector mechanism 103 and thereby mechanically decouples the conductive connector mechanism 103 from skin. The supportive textile 401 is also configured to mechanically support the apparatus 100.

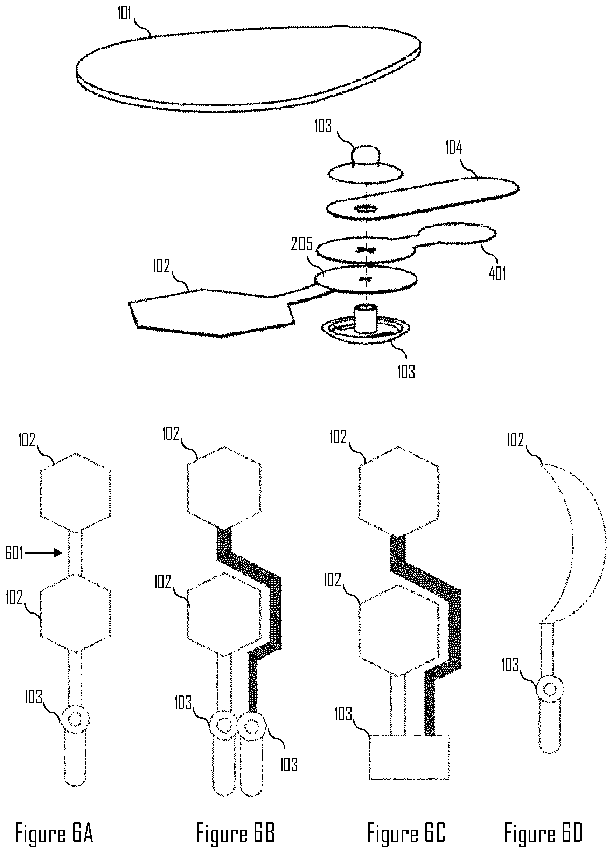

Figure 5 shows an example of the apparatus 100 in an exploded view according to a yet further example embodiment.

In the example of Figure 5, the apparatus 100 comprises conductive textile 102 for contacting skin, a holding layer 101 for holding the conductive textile 102 in contact with skin, a conductive connector mechanism 103 operatively connected to the conductive textile 102, conductive polymer layer 205 provided on top of the conductive textile 102, a cable detaching mechanism 104 and supportive textile 401. In the example of Figure 5, the conductive polymer layer 205 comprises shielding properties. However, the conductive polymer layer 205 may also be used for other purposes than shielding.

According to an example embodiment, the conductive polymer layer 205 may be provided under the conductive textile 102 such that the conductive polymer layer 205 is configured to contact skin. In such an embodiment, the conductive polymer layer 205 comprises highly conductive particles in order to provide a conductive interface between skin and the conductive textile 102. During manufacturing the conductive polymer layer 205 also protects the conductive textile 102 such that the conductive polymer layer 205 prevents melt substance such as silicone or thermoplastic elastomer from flowing through the conductive textile 102.

As the conductive polymer layer 205 is tacky, the conductive polymer layer 205 under the conductive textile 102 increases the electrical contact with skin thereby improving signal quality. In such an embodiment, the conductive textile 102 may comprise a mesh size suitable for allowing viscous conductive polymer to flow through the conductive textile 102 and thereby mechanically attach the conductive polymer layer 205 to the conductive textile 102 and the conductive textile 102 to the holding layer 101. A conductive polymer layer 205 provided under the conductive textile 102 provides conductivity between skin and the conductive textile 102. The conductive textile 102 further provides conductivity in the direction parallel to skin. The conductive textile may be used also as a supporting structure keeping conductive particles close to each other even when stretching the apparatus 100. Hence, the durability of the apparatus 100 may be improved.

According to an example embodiment, the apparatus 100 comprises a plurality of electrodes. An electrode may comprise conductive textile 102.

Figure 6A shows an example arrangement of the apparatus 100 comprising a first electrode 102 and a second electrode 102 in form of two pieces of conductive textile. In the example of Figure 6A, the first electrode 102 and the second electrode 102 are connected to a measuring device using a mechanical conductive connector mechanism 103 such as a snap joint. The first electrode 102 and the second electrode 102 are mechanically and electrically separated by a holding layer. Further, the mechanical parts between the first electrode 102 and the second electrode 102 are mechanically and electrically separated from skin by the holding layer. In the example of Figure 6, the distance 601 between the first electrode 102 and the second electrode 102 is determined based on the application of the apparatus 100. For example, for ECG the distance 601 may be, for example, 8 - 12 cm, while for EEG and EMG it may be shorter.

Figure 6B shows another example arrangement of the apparatus 100 comprising a first electrode 102 and a second electrode 102 in form of two pieces of conductive textile. In the example of Figure 6B, the first electrode 102 and the second electrode 102 are connected to a measuring device using a first mechanical conductive connector mechanism 103 and a second mechanical conductive connector mechanism 103 such as a snap joint. The first electrode 102 and the second electrode 102 are mechanically and electrically separated by a holding layer. Further, the mechanical parts connecting the first electrode 102 and the second electrode 102 to a measurement device are mechanically and electrically separated from skin by the holding layer.

Figure 6C shows a further example arrangement of the apparatus 100 comprising a first electrode 102 and a second electrode 102 in form of two pieces of conductive textile. In the example of Figure 6C, the first electrode 102 and the second electrode 102 are connected to a measuring device using a common conductive connector mechanism 103. In the example of Figure 6C the conductive connector mechanism 103 may comprise, for example, a ribbon cable or a wireless connection such as a wireless transmitter. The first electrode 102 and the second electrode 102 are mechanically and electrically separated by a holding layer. Further, the mechanical parts connecting the first electrode 102 and the second electrode 102 to a measuring device are mechanically and electrically separated from skin by the holding layer.

Figure 6D shows a yet further example arrangement of the apparatus 100 comprising an electrode 102 in form of a of conductive textile. In the example of Figure 6C, the electrode 102 has a shape suitable for measuring bio-signals such as EEG behind an ear. The electrode in the example of Figure 6D may be combined with electrodes presented in Figures 6A, 6B and/or 6C and conductive connector mechanisms 103 presented in Figures 6A, 6B and/or 6C.

Without limiting the scope of the claims, an advantage of the apparatus 100 is that it does not require electrode gel. Another advantage is that the apparatus 100 is flexible and configured to reduce motion artifact.

Without in any way limiting the scope, interpretation, or application of the claims appearing below, a technical effect of one or more of the example embodiments disclosed herein is that skin irritations may be reduced and the time of use may be extended as there is no need to add electrode gel when using the apparatus 100. Another technical effect is improved signal quality due to reduced motion artifact and skin conformal, electrically conductive materials.

It will be obvious to a person skilled in the art that, as the technology advances, the inventive concept can be implemented in various ways. The invention and its embodiments are not limited to the examples described above but may vary within the scope of the claims.

## Claims

1. An apparatus for measuring bio-signals, the apparatus comprising:
conductive textile for contacting skin;
a holding layer for holding the conductive textile in contact with skin;
and
a conductive connector mechanism operatively connected to the conductive textile, wherein the conductive connector mechanism is integrated in the holding layer.

2. The apparatus according to claim 1, wherein the holding layer comprises a non-conductive polymer holding layer.

3. The apparatus according to claim 2, wherein the holding layer comprises silicone or thermoplastic elastomer.

4. The apparatus according to any preceding claim, wherein conductive textile is at least partially integrated in the holding layer.

5. The apparatus according to any preceding claim, wherein the holding layer comprises a first part and a second part.

6. The apparatus according to any preceding claim, wherein the holding layer is hydrophobic.

7. The apparatus according to any preceding claim, wherein holding layer is a moulded layer.

8. The apparatus according to according to any preceding claim, wherein the conductive connector mechanism comprises a snap joint.

9. The apparatus according to according to any preceding claim, wherein the conductive textile comprises silver coated textile.

10. The apparatus according to according to any preceding claim, wherein the shape of the conductive textile comprises a polygon.

11. The apparatus according to any preceding claim, wherein the apparatus further comprises a cable detaching mechanism.

12. The apparatus according to any preceding claim, wherein the apparatus further comprises supportive textile.

13. The apparatus according to any preceding claim, wherein the apparatus comprises a dry surface electrode.
